(19) Europäisches Patentamt
European Patent Office
Office européen des brevets

(11) EP 2 618 801 B2

(12) **NEW EUROPEAN PATENT SPECIFICATION**
After opposition procedure

(45) Date of publication and mention of the opposition decision:
**20.01.2021 Bulletin 2021/03**

(45) Mention of the grant of the patent:
**20.06.2018 Bulletin 2018/25**

(21) Application number: **11764148.0**

(22) Date of filing: **23.09.2011**

(51) Int Cl.:
*A61Q 5/12* (2006.01) *A61Q 5/00* (2006.01)
*A61K 8/20* (2006.01) *A61K 8/365* (2006.01)
*A61K 8/34* (2006.01) *A61K 8/36* (2006.01)

(86) International application number:
**PCT/EP2011/066601**

(87) International publication number:
**WO 2012/038536 (29.03.2012 Gazette 2012/13)**

(54) **COSMETIC COMPOSITION COMPRISING AT LEAST ONE HYGROSCOPIC SALT, AT LEAST ONE AROMATIC POLYOL ETHER AND AT LEAST ONE DIOL, AND COSMETIC TREATMENT PROCESS**

KOSMETISCHE ZUSANMENSETZUNG ENTHALTEND MINDESTENS EIN HYGROSKOPISCHES SALZ, EIN AROMATISCHES POLYOL ETHER UND EIN DIOL.

COMPOSITION COSMÉTIQUE COMPORTANT AU MOINS UN SEL HYGROSCOPIQUE, UN ÉTHER DE POLYOL AROMATIQUE ET UN DIOL, ET PROCÉDÉ DE TRAITEMENT COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2010 US 390280 P**
**24.09.2010 FR 1057712**

(43) Date of publication of application:
**31.07.2013 Bulletin 2013/31**

(73) Proprietor: **L'Oréal**
**75008 Paris (FR)**

(72) Inventors:
- **ALBERT, Anne-Sophie**
  **F-75009 Paris (FR)**
- **PLOS, Grégory**
  **F-75018 Paris (FR)**

(74) Representative: **Martin-Charbonneau, Virginie**
**Casalonga & Partners**
**Bayerstrasse 71/73**
**80335 München (DE)**

(56) References cited:
**WO-A1-2010/086695** **DE-U1- 9 210 516**
**JP-A- S6 087 208** **JP-A- 2003 095 891**
**US-A1- 2005 189 377** **US-A1- 2007 010 408**
**US-A1- 2007 054 967** **US-A1- 2009 104 136**
**US-B1- 6 284 230**

- **"Truth In Aging - Eyelashes & Brows", , 11 January 2010 (2010-01-11), XP002648640, Retrieved from the Internet: URL:http://truthinaging.com/eyes/eyelash-growth-products-do-they-work-and-are-they-safe-2010-update [retrieved on 2011-07-08]**
- **DATABASE GNPD [Online] Mintel; 10 February 2009 (2009-02-10), "Nature'sStyle Conditioner-For Dry, Damaged Hair", XP002648641, Database accession no. 93035**

Remarks:
The file contains technical information submitted after the application was filed and not included in this specification

EP 2 618 801 B2

## Description

The present invention relates to a cosmetic composition for treating keratin fibres, in particular human keratin fibres such as the hair, comprising, in a cosmetically acceptable medium, one or more polyol ethers containing an aromatic group, one or more non-aromatic and non-etherified $C_6$-$C_8$ diols and one or more hygroscopic water soluble salts in a content of at least 1% by weight relative to the total weight of the cosmetic composition. The present invention also relates to a cosmetic process for treating keratin fibres, and also to a use employing the said cosmetic composition.

Hair is generally damaged and embrittled by the action of external atmospheric agents such as light and bad weather, and also by mechanical or chemical treatments, such as brushing, combing, dyeing, bleaching, permanent-waving and/or relaxing.

Hair is thus damaged by these various factors and may over time become dry, coarse or dull, especially in fragile areas, and more particularly at the ends. Moreover, high relative humidity of the air usually leads to swelling of the head of hair and to the appearance of frizziness, which makes the head of hair increasingly difficult to manage.

Thus, to overcome these drawbacks, it is common practice to resort to haircare products using compositions that condition the hair appropriately, giving it satisfactory cosmetic properties, especially in terms of smoothness, sheen, softness, suppleness, lightness, a natural feel or ease of disentangling, while at the same time controlling the volume given to the head of hair.

These haircare compositions may be, for example, conditioning shampoos, hair conditioners, masks or sera, and may be in the form of gels, hair lotions or care creams that are more or less thick.

These compositions may comprise silicones, which are used as conditioning agents, so as to give the hair a satisfactory level of care and good control of the volume of the head of hair.

However, silicones often have the drawback of making the hair very lank and heavy, which leads to the phenomenon commonly known as the "build-up effect". In other words, silicones become deposited in a large amount of material, which has the consequence of making the head of hair lank and of limiting the use of silicones for conditioning the hair.

Moreover, it is also known practice in the care field to use cosmetic compositions containing hygroscopic salts.

Specifically, international patent application WO 95/01152 describes a conditioning shampoo composition comprising at least one surfactant chosen from anionic, nonionic, amphoteric and zwitterionic surfactants, at least one non-volatile conditioning agent chosen from insoluble silicones, water-soluble organic cationic compounds such as cationic surfactants and cationic polymers, and salts of polyvalent cationic metal.

Similarly, Japanese patent application JP 2003/095 891 concerns a rinse-out hair composition intended to be used as a hair conditioner, containing at least one long-chain fatty alcohol, a quaternary ammonium salt bearing at least one chain formed from 8 to 24 carbon atoms and at least one water-soluble organic and/or inorganic salt, the pH of the composition at 30°C being between 5 and 6.5.

Japanese patent application JP 2000/191 514 describes a composition for cleansing sebum, the surface tension of which, relative to oleic acid at 25°C, ranges from 0 to 0.05 mN/m, containing a mineral salt, a nonionic surfactant and a cationic surfactant.

In the field of cosmetic skincare, Japanese patent application JP 200247119 relates to a cleansing and moisturizing cosmetic composition containing $Mg^{2+}$ and $Ca^{2+}$ ions, the ratio [Mg]/[Ca] being between 0.2 and 3.6 and the total amount of Ca and Mg ions being from 0.052 to 4.2 mol/L.

Finally, Japanese patent application JP 01233208 relates to a treatment composition containing water-soluble substances such as inorganic salts, which create insoluble substances within the hair.

Cosmetic compositions based on hygroscopic salts generally contain chemical preserving agents due to their high water content and to the presence in these compositions of components such as vitamins and amino acids. The reason for this is that these components may especially promote the growth of microorganisms, which means that these compositions are liable to be contaminated during their manufacture, but also during their use by the consumer, in particular when products in jars are touched with the fingers. Thus, chemical preserving agents protect compositions containing hygroscopic salts against the microorganisms that are liable to grow inside the compositions at the time of their manufacture and those that the user might introduce therein when handling them.

However, the chemical preserving agents that are conventionally used, such as benzoic acid, have a tendency to inhibit the effects associated with hygroscopic salts and to degrade the cosmetic properties and also the maintenance of the volume of the head of hair that might be imparted by such compositions.

There is thus a real need to develop cosmetic compositions, for example hair conditioners, that do not have the combination of drawbacks described above, i.e. which are capable of controlling the volume of the head hair and of conditioning the hair, especially by affording it softness, uniformity, sheen, suppleness, lightness, a natural feel and good capacity to be straightened, while at the same time being able to be conserved efficiently.

The Applicant has discovered, surprisingly, that it is possible to formulate compositions for the cosmetic treatment of keratin fibres, which have the desired properties, by combining in these compositions one or more polyol ethers

containing an aromatic group, one or more non-aromatic and non-etherified $C_6$-$C_8$ diols and one or more hygroscopic water soluble salts, in a particular content.

Specifically, it has been found that such a combination can give keratin fibres, and more particularly the hair, improved cosmetic properties especially as regards the level of sheen, the smoothness, the suppleness, the lightness, the feel, the uniformity and the fineness, while at the same time controlling the volume of the hair.

In particular, on wet hair, smoother, more supple, finer hair is especially obtained. Furthermore, once dried, hair treated with the compositions according to the invention is especially smoother, shinier, finer and lighter, and has a more silky feel.

Moreover, the compositions according to the invention lead to better control of the swelling of the head of hair, and can do so for various relative humidities, ranging in particular from 20% to 80%, which enables the hairstyle to remain in place more easily, allows better control of the volume of the hair and greatly reduces the frizziness. Thus, the compositions according to the invention facilitate the shaping of hair.

The term "relative humidity" represents the measurement of the amount of water in the air. This measurement is taken using a machine such as a psychrometer or a capacitive-probe hygrometer, and is defined by the ratio of the pressure exerted by the water vapour contained in the air at a given temperature to the saturating vapour pressure (i.e. the maximum amount of water vapour that this air could absorb at this temperature). It is given as a percentage on a scale ranging from 0 to 100%.

Thus, it has been found that the combination of a polyol ether containing an aromatic group and a non-aromatic and non-etherified $C_6$-$C_8$ diol can prevent degradation of the cosmetic properties and maintain the volume of the head hair imparted by compositions containing hygroscopic salts, and can do so whilst ensuring efficient microbiological conservation.

In other words, the cosmetic compositions according to the invention withstand contamination by microorganisms.

The compositions according to the invention are also stable on storage, both at room temperature (20-25°C) and at 45°C, especially as regards their visual aspect and their viscosity. The cosmetic compositions obtained also have good working properties, i.e. these compositions can be rinsed out easily and be spread more easily over the entire head of hair.

Finally, hair treated with the cosmetic compositions according to the invention also dries more quickly.

The present invention relates especially to a cosmetic composition for treating keratin fibres, in particular human keratin fibres such as the hair, comprising:

- one or more polyol ethers containing one or more aromatic groups chosen from ethylene glycol monophenyl ether (or phenoxyethanol), ethylene glycol diphenyl ether, ethylene glycol monobenzyl ether, propylene glycol monophenyl ether, propylene glycol monobenzyl ether, diethylene glycol monophenyl ether, diethylene glycol monobenzyl ether, dipropylene glycol monophenyl ether and dipropylene glycol monobenzyl ether, and mixtures thereof, in a content ranging from 0.05 to 2% by weight relative to the total weight of the composition,
- one or more non-aromatic and non-etherified $C_6$-$C_8$ diols in a content ranging from 0.05 to 2% by weight relative to the total weight of the composition and
- one or more hygroscopic water-soluble salts in a content ranging from 2 to 10% by weight, relative to the total weight of the said cosmetic composition; the hygroscopic salt being calcium chloride.

The present invention also relates to a cosmetic process for treating the hair, comprising the application to the said fibres of the composition according to the invention.

The invention also relates to the use of the composition according to the invention as a shampoo or as a hair conditioner, preferably as a hair conditioner.

Other subjects and characteristics, aspects and advantages of the invention will emerge even more clearly on reading the description and the example that follows. More preferentially, the polyol ether containing one or more aromatic groups used in the cosmetic composition according to the invention is ethylene glycol monophenyl ether (or phenoxyethanol).

The cosmetic composition according to the invention also contains one or more non-aromatic and non-etherified $C_6$-$C_8$ diols.

For the purposes of the present invention, the term "non-aromatic and non-etherified $C_4$-$C_{10}$ diol" means any hydrocarbon-based compound comprising two hydroxyl functions and from six to eight carbon atoms and not comprising any aromatic groups or any ether groups in its structure.

The non-aromatic and non-etherified $C_4$-$C_{10}$ diol(s) used in the cosmetic composition according to the invention may be chosen from, hexylene glycol, 1,2-hexanediol, 1,6-hexanediol and 1,2-octanediol (or caprylyl glycol), and mixtures thereof.

Preferably, the diol is a vicinal diol, i.e. borne by two neighbouring, preferably adjacent, carbon atoms. Even

more preferentially, the diol is a 1,2-diol.

In one preferred variant, the non-aromatic and non-etherified $C_6$-$C_8$ diol used in the cosmetic composition according to the invention is 1,2-octanediol.

Preferably, the total content of polyol ethers containing an aromatic group and of non-aromatic and non-etherified $C_6$-$C_8$ diols ranges from 0.1% to 2% by weight relative to the total weight of the cosmetic composition.

The cosmetic composition also comprises one or more hygroscopic water-soluble salts in a content ranging from 2 to 10% by weight, relative to the total weight of the said cosmetic composition; the hygroscopic salt being calcium chloride. For the purposes of the present invention, the term "hygroscopic salt" means a salt that is capable of absorbing water from the surrounding atmosphere.

The hygroscopic salt(s) used in the cosmetic composition according to the invention absorb at least 0.05 g of water per gram of salt at 25°C, at 25% relative humidity and at atmospheric pressure.

The hygroscopic salts of the invention are water-soluble.

For the purposes of the present invention, the term "water-soluble salt" means a salt with a solubility of greater than or equal to 20 g/litre (g/L) in water measured at 20°C, preferentially a solubility of greater than or equal to 100 g/L and even more preferentially a solubility of greater than or equal to 500 g/L.

The cosmetic composition according to the invention may also comprise one or more surfactants selected from cationic, anionic, nonionic, amphoteric and zwitterionic surfactants.

For the purposes of the present invention, the term "cationic surfactant" means a surfactant that is positively charged when it is contained in the composition according to the invention. This surfactant may bear one or more positive permanent charges or may contain one or more cationizable functions in the composition according to the invention.

As examples of cationic surfactants that may be used in the cosmetic composition, mention may be made especially of optionally polyoxyalkylenated primary, secondary or tertiary fatty amines, or salts thereof, and quaternary ammonium salts, and mixtures thereof.

The fatty amines generally comprise at least one $C_8$-$C_{30}$ hydrocarbon-based chain. Among the fatty amines that may be used according to the invention, an example that may be mentioned is stearylamidopropyldimethylamine.

Examples of quaternary ammonium salts that may especially be mentioned include:

- those that have the general formula (I) below:

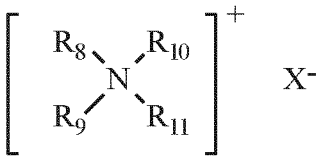

(I)

in which the radicals $R_8$ to $R_{11}$, which may be identical or different, represent a linear or branched aliphatic radical comprising from 1 to 30 carbon atoms or an aromatic radical such as aryl or alkylaryl, at least one of the radicals $R_8$ to $R_{11}$ comprising from 8 to 30 carbon atoms and preferably from 12 to 24 carbon atoms. The aliphatic radicals may comprise heteroatoms especially such as oxygen, nitrogen, sulfur and halogens.
The aliphatic radicals are, for example, chosen from alkyl, alkoxy, polyoxy($C_2$-$C_6$)alkylene, alkylamide, ($C_{12}$-$C_{22}$)alkylamido($C_2$-$C_6$)alkyl, ($C_{12}$-$C_{22}$)alkyl acetate or hydroxyalkyl radicals comprising approximately from 1 to 30 carbon atoms; $X^-$ is an anion chosen from the group of halides, phosphates, acetates, lactates, ($C_2$-$C_6$)alkyl sulfates, or alkyl- or alkylaryl-sulfonates;

- quaternary ammonium salts of imidazoline, for instance those of formula (II) below:

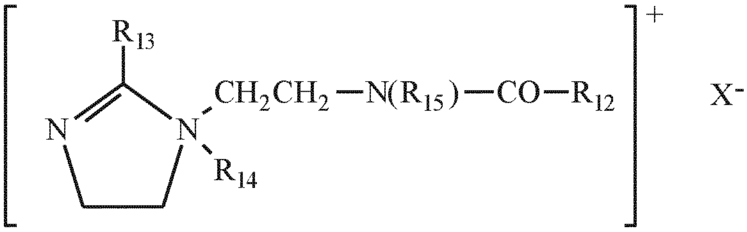

(II)

in which $R_{12}$ represents an alkenyl or alkyl radical comprising from 8 to 30 carbon atoms, for example derived from tallow fatty acids, $R_{13}$ represents a hydrogen atom, a $C_1$-$C_4$ alkyl radical or an alkenyl or alkyl radical comprising from 8 to 30 carbon atoms, $R_{14}$ represents a $C_1$-$C_4$ alkyl radical, $R_{15}$ represents a hydrogen atom or a $C_1$-$C_4$ alkyl

radical and $X^-$ is an anion chosen from the group of the halides, phosphates, acetates, lactates, alkyl sulfates, or alkyl- or alkylaryl-sulfonates. Preferably, $R_{12}$ and $R_{13}$ denote a mixture of alkenyl or alkyl radicals comprising from 12 to 21 carbon atoms, for example fatty acid derivatives of tallow, $R_{14}$ denotes a methyl radical and $R_{15}$ denotes a hydrogen atom. Such a product is sold, for example, under the name Rewoquat® W 75 by the company Rewo;

- di- or triquaternary ammonium salts, in particular of formula (III):

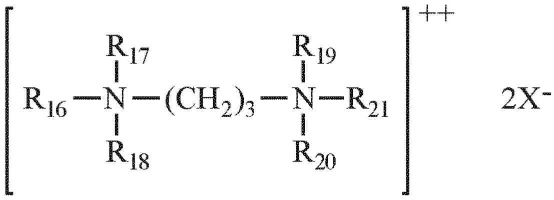

(III)

in which $R_{16}$ denotes an alkyl radical containing approximately from 16 to 30 carbon atoms, which is optionally hydroxylated and/or interrupted with one or more oxygen atoms and optionally branched, $R_{17}$ is selected from an alkyl radical containing from 1 to 4 carbon atoms or a group $(R_{16a})(R_{17a})(R_{18a})N^+\text{-}(CH_2)_3\text{-}$, $R_{16a}$, $R_{17a}$, $R_{18a}$, $R_{18}$, $R_{19}$, $R_{20}$ and $R_{21}$, which may be identical or different, are selected from hydrogen and an alkyl radical containing from 1 to 4 carbon atoms, and $X^-$ is an anion selected from the group of halides, acetates, phosphates, nitrates and methyl sulfates. Such compounds are, for example, Finquat CT-P, available from the company Finetex (Quaternium 89), and Finquat CT, available from the company Finetex (Quaternium 75),

- quaternary ammonium salts containing at least one ester function, such as those of formula (IV) below:

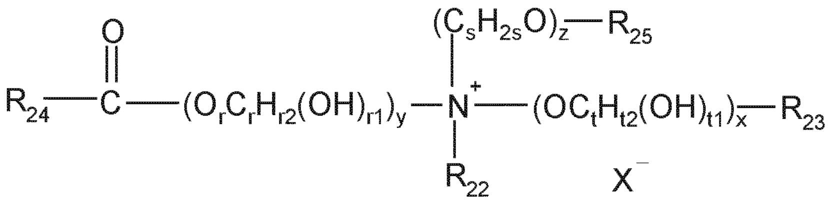

(IV)

in which:

$R_{22}$ is chosen from $C_1$-$C_6$ alkyl and $C_1$-$C_6$ hydroxyalkyl or dihydroxyalkyl radicals;
$R_{23}$ is chosen from:
the radical

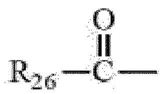

- radicals $R_{27}$, which are linear or branched, saturated or unsaturated $C_1$-$C_{22}$ hydrocarbon-based radicals,
- a hydrogen atom,

$R_{25}$ is chosen from:

- the radical

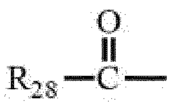

- radicals R, which are linear or branched, saturated or unsaturated $C_1$-$C_6$ hydrocarbon-based radicals,
- a hydrogen atom,

$R_{24}$, $R_{26}$ and $R_{28}$, which may be identical or different, are chosen from linear or branched, saturated

or unsaturated $C_7$-$C_{21}$ hydrocarbon-based radicals;
r, s and t, which may be identical or different, are integers ranging from 2 to 6;
y is an integer ranging from 1 to 10;
x and z, which may be identical or different, are integers ranging from 0 to 10;
r1 or t1 = 0 or 1,

$$r2 + r1 = 2r,$$

$$t2 + t1 = 2t,$$

$X^-$ is a simple or complex, organic or mineral anion; with the proviso that the sum x + y + z is from 1 to 15, that when x is 0, then $R_{23}$ denotes $R_{27}$ and that when z is 0, then $R_{25}$ denotes $R_{29}$.

The alkyl radicals $R_{22}$ may be linear or branched, but more particularly linear.
$R_{22}$ preferably denotes a methyl, ethyl, hydroxyethyl or dihydroxypropyl radical, and more particularly a methyl or ethyl radical.
Advantageously, the sum x + y + z is from 1 to 10.
When $R_{23}$ is a hydrocarbon-based radical $R_{27}$, it may be long and may contain from 12 to 22 carbon atoms, or may be short and may contain from 1 to 3 carbon atoms.
When $R_{25}$ is a hydrocarbon-based radical $R_{29}$, it preferably contains 1 to 3 carbon atoms.
Advantageously, $R_{24}$, $R_{26}$ and $R_{28}$, which may be identical or different, are chosen from linear or branched, saturated or unsaturated $C_{11}$-$C_{21}$ hydrocarbon-based radicals, and more particularly from linear or branched, saturated or unsaturated $C_{11}$-$C_{21}$ alkyl and alkenyl radicals.
Preferably, x and z, which may be identical or different, are equal to 0 or 1.
Advantageously, y is equal to 1.
Preferably, r, s and t, which may be identical or different, are equal to 2 or 3, and even more particularly are equal to 2.
The anion $X^-$ is preferably a halide (chloride, bromide or iodide) or an alkyl sulfate, more particularly methyl sulfate. It is possible, however, to use methanesulfonate, phosphate, nitrate or tosylate, an anion derived from organic acid, such as acetate or lactate, or any other anion that is compatible with ester-functional ammonium.
The anion $X^-$ is even more particularly chloride or methyl sulfate.
Use is made more particularly, in the composition according to the invention, of the ammonium salts of formula (IV) in which:

- $R_{22}$ denotes a methyl or ethyl radical,
- x and y are equal to 1;
- z is equal to 0 or 1;
- r, s and t are equal to 2;
- $R_{23}$ is chosen from:

- the radical

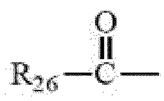

- methyl, ethyl or $C_{14}$-$C_{22}$ hydrocarbon-based radicals,
- a hydrogen atom;

- $R_{25}$ is chosen from:

- the radical

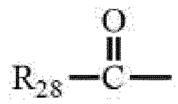

- a hydrogen atom;

- $R_{24}$, $R_{26}$ and $R_{28}$, which may be identical or different, are chosen from linear or branched, saturated or unsaturated $C_{13}$-$C_{17}$ hydrocarbon-based radicals and preferably from linear or branched, saturated or unsaturated $C_{13}$-$C_{17}$ alkyl and alkenyl radicals.

The hydrocarbon-based radicals are advantageously linear. Examples that may be mentioned include the compounds of formula (IV) such as the diacyloxyethyldimethylammonium, diacyloxyethylhydroxyethylmethylammonium, monoacyloxyethyldihydroxy ethyl methyl ammonium, triacyloxyethylmethylammonium and monoacyloxyethylhydroxyethyldimethylammonium salts (chloride or methyl sulfate in particular), and mixtures thereof. The acyl radicals preferably contain 14 to 18 carbon atoms and are obtained more particularly from a plant oil such as palm oil or sunflower oil. When the compound contains several acyl radicals, these radicals may be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by a quaternization using an alkylating agent such as an alkyl halide (preferably a methyl or ethyl halide), a dialkyl sulfate (preferably dimethyl or diethyl sulfate), methyl methanesulfonate, methyl para-toluenesulfonate, glycol chlorohydrin or glycerol chlorohydrin.

Such compounds are, for example, sold under the names Dehyquart® by Henkel, Stepanquat® by Stepan, Noxamium® by Ceca or Rewoquat® WE 18 by Rewo-Witco.

The composition according to the invention preferably contains a mixture of quaternary ammonium salts of mono-, di- and triesters with a weight majority of diester salts.

Examples of mixtures of ammonium salts that may be used include the mixture containing 15% to 30% by weight of acyloxyethyldihydroxyethylmethylammonium methyl sulfate, 45% to 60% of diacyloxyethylhydroxyethylmethylammonium methyl sulfate and 15% to 30% of triacyloxyethylmethylammonium methyl sulfate, the acyl radicals containing from 14 to 18 carbon atoms and being derived from palm oil that is optionally partially hydrogenated.

It is also possible to use the ammonium salts containing at least one ester function that are described in patents US-A-4 874 554 and US-A-4 137 180.

Preferably, the cosmetic composition according to the invention comprises one or more cationic surfactants chosen from the quaternary ammonium salts corresponding to formula (I).

Preference is given, among the quaternary ammonium salts of formula (VIII), on the one hand, to tetraalkylammonium chlorides, such as, for example, dialkyldimethylammonium or alkyltrimethylammonium chlorides in which the alkyl radical comprises approximately from 12 to 22 carbon atoms, in particular behenyltrimethylammonium chloride, distearyldimethylammonium chloride, cetyltrimethylammonium chloride or benzyldimethylstearylammonium chloride, or also, on the other hand, to distearoyl ethyl hydroxy ethyl methyl ammonium methosulfate, dipalmitoylethylhydroxyethylammonium methosulfate or distearoylethylhydroxyethylammonium methosulfate, or also, finally, to palmitylamidopropyltrimethylammonium chloride or stearamidopropyldimethyl(myristyl acetate)ammonium chloride, sold under the name Ceraphyl® 70 by Van Dyk.

Among all the cationic surfactants that may be present in the composition according to the invention, cationic surfactants from among cetyltrimethylammonium (INCI: cetrimonium), behenyltrimethylammonium (INCI: behentrimonium), dipalmitoylethylhydroxy ethylmethyl ammonium, distearoylethylhydroxy ethylmethylammonium, methyl($C_9$-$C_{19}$) alkyl($C_{10}$-$C_{20}$)alkylamidoethylimidazolium and stearamidopropyldimethylamine salts (chloride or methosulfate), and the stearamidopropyldimethylammonium salt, and mixtures thereof, are preferably chosen.

Use may be made of behenoylhydroxypropyltrimethylammonium chloride sold by KAO under the name Quatarmin BTC 131.

When they are present, the amount of the cationic surfactant(s) preferably ranges from 0.01% to 20% by weight and better still from 0.1% to 5% by weight relative to the total weight of the composition.

The term "anionic surfactant" means a surfactant comprising, as ionic or ionizable groups, only anionic groups. These anionic groups are preferably chosen from $CO_2H$, $CO_2^-$, $SO_3H$, $SO_3^-$, $OSO_3H$, $OSO_3^-$, $H_2PO_3$, $HPO_3^-$, $PO_3^{2-}$, $H_2PO_2$, $HPO_2$, $HPO_2^-$, $PO_2^-$, POH and $PO^-$ groups.

As examples of anionic surfactants that may be used in the composition according to the invention, mention may be made of alkyl sulfates, alkyl ether sulfates, alkylamido ether sulfates, alkylarylpolyether sulfates, monoglyceride sulfates, alkylsulfonates, alkylamidesulfonates, alkylarylsulfonates, $\alpha$-olefin sulfonates, paraffin sulfonates, alkylsulfosuccinates, alkylether sulfosuccinates, alkylamide sulfosuccinates, alkylsulfoacetates, acylsarcosinates, acylglutamates, alkylsulfosuccinamates, acylisethionates and N-acyltaurates, salts of alkyl monoesters of polyglycoside-polycarboxylic acids, acyllactylates, D-galactoside-uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds comprising from 6 to 24 carbon atoms and the aryl group denoting a phenyl group.

These compounds may be oxyethylenated and then preferably comprise from 1 to 50 ethylene oxide units.

The salts of $C_{6-24}$ alkyl monoesters and polyglycoside-polycarboxylic acids may be selected from $C_{6-24}$ alkyl polyglycoside-citrates, $C_{6-24}$ alkyl polyglycoside-tartrates and $C_{6-24}$ alkyl polyglycoside-sulfosuccinates.

When the anionic surfactant(s) (ii) are in salt form, they may be chosen from alkali metal salts such as the sodium or potassium salt and preferably the sodium salt, the ammonium salts, the amine salts and in particular amino alcohol salts or the alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)-aminomethane salts.

Alkali metal or alkaline-earth metal salts, and in particular sodium or magnesium salts, are preferably used.

Among the anionic surfactants, it is preferred, according to the invention, to use alkyl sulfate salts and alkyl ether sulfate salts and mixtures thereof.

When they are present, the amount of the anionic surfactant(s) is preferably within the range from 0.1% to 50% by weight, better still from 1% to 30% by weight and even better still from 4% to 20% by weight relative to the total weight of the composition.

Even more preferentially, the composition does not contain any anionic surfactants.

Examples of additional nonionic surfactants that may be used in the compositions of the present invention are described, for example, in the Handbook of Surfactants by M.R. Porter, published by Blackie & Son (Glasgow and London), 1991, pp. 116-178. They are especially chosen from polyethoxylated, polypropoxylated or polyglycerolated alcohols, $\alpha$-diols and (C1-20)alkylphenols, containing at least one fatty chain comprising, for example, from 8 to 18 carbon atoms, the number of ethylene oxide or propylene oxide groups possibly ranging especially from 2 to 50, and the number of glycerol groups possibly ranging especially from 2 to 30.

Mention may also be made of condensates of ethylene oxide and of propylene oxide with fatty alcohols; polyethoxylated fatty amides preferably having from 2 to 30 ethylene oxide units, polyglycerolated fatty amides containing on average 1 to 5, and in particular 1.5 to 4, ethoxy related fatty acid esters of sorbitan containing from 2 to 30 ethylene oxide units, fatty acid esters of sucrose, fatty acid esters of polyethylene glycol, ($C_{6-24}$ alkyl)polyglycosides, N-($C_{6-24}$ alkyl)glucamine derivatives, amine oxides such as ($C_{10}C_{14}$)alkylamine oxides or N($C_{10-14}$ acyl)aminopropylmorpholine oxides.

When they are present, the amount of the additional nonionic surfactant(s) is preferably within the range from 0.1% to 30% by weight and better still from 0.2% to 20% by weight relative to the total weight of the composition.

The amphoteric or zwitterionic surfactant(s) that may be used in the present invention may especially be optionally quaternized, secondary or tertiary aliphatic amine derivatives, in which the aliphatic group is a linear or branched chain containing from 8 to 22 carbon atoms, the said amine derivatives containing at least one anionic group such as, for example, a carboxylate, sulfonate, sulfate, phosphate or phosphonate group. Mention may be made in particular of ($C_8$-$C_{20}$)alkylbetaines, sulfobetaines, ($C_8$-$C_{20}$alkyl)amido($C_{3-8}$alkyl)betaines and ($C_8$-$C_{20}$alkyl)amido($C_6$-$C_8$ alkyl)sulfobetaines. Among the optionally quaternized secondary or tertiary aliphatic amine derivatives that can be used, as defined above, mention may also be made of the compounds of respective structures (A1) and (A2) below:

$$Ra\text{-}CONHCH_2CH_2\text{-}N^+(Rb)(Rc)(CH_2COO^-) \qquad (A1)$$

in which:

Ra represents a $C_{10}$-$C_{30}$ alkyl or alkenyl group derived from an acid
Ra-COOH, preferably present in hydrolysed coconut oil, a heptyl, nonyl or undecyl group,
Rb represents a $\beta$-hydroxyethyl group, and
Rc represents a carboxymethyl group; and

$$Ra'\text{-}CONHCH_2CH_2\text{-}N(B)(B') \qquad (A2)$$

in which:

B represents $-CH_2CH_2OX'$,
B' represents $-(CH_2)z-Y'$, with z = 1 or 2,
X' represents the group $-CH_2-COOH$, $CH_2-COOZ'$, $-CH_2CH_2-COOH$, $-CH_2CH_2-COOZ'$, or a hydrogen atom,
Y' represents -COOH, -COOZ', the group $-CH_2-CHOH-SO_3$ H or $-CH_2-CHOH-SO_3$ Z',
Z' represents an ion derived from an alkali metal or alkaline-earth metal, such as sodium, an ammonium ion or an ion derived from an organic amine.
Ra' represents a $C_{10}$-$C_{30}$ alkyl or alkenyl group of an acid Ra'-COOH which is preferably present in coconut oil or in hydrolysed linseed oil, or an alkyl group, especially a $C_{17}$ group, and its iso form, or an unsaturated $C_{17}$ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.
By way of example, mention may be made of the cocoamphodiacetate sold by the company Rhodia under the trade name Miranol® C2M Concentrate.
Among the amphoteric or zwitterionic surfactants mentioned above that are preferably used are ($C_{8-20}$ alkyl)betaines and ($C_{8-20}$ alkyl)amido($C_{6-8}$ alkyl)betaines such as cocamidopropylbetaine, and mixtures thereof.
When they are present, the amount of the amphoteric or zwitterionic surfactant(s) is preferably within the range from 0.1% to 20% by weight and better still from 0.2% to 10% by weight relative to the total weight of the composition.
Preferably, the cosmetic composition comprises one or more cationic surfactants.
Even more preferentially, the cationic surfactant(s) that may be used in the cosmetic composition according to the invention are chosen from the compounds of formula (I) or (IV) and more particularly from cetyltrimethylammonium and behenyltrimethylammonium salts.
The cosmetic composition according to the invention may also comprise one or more silicones.
The silicone(s) that may be present in the composition according to the invention are in particular polyorganosiloxanes that may be in the form of aqueous solutions, i.e. dissolved, or optionally in the form of dispersions or microdispersions, or of aqueous emulsions. The polyorganosiloxanes may also be in the form of oils, waxes, resins or gums.
Organopolysiloxanes are defined in greater detail in Walter Noll's Chemistry and Technology of Silicones (1968), Academic Press.
The silicone(s) may be volatile or non-volatile.
When they are volatile, the silicones are more particularly chosen from those with a boiling point of between 60°C and 260°C, and even more particularly from:

(i) cyclic silicones comprising from 3 to 7 and preferably 4 to 5 silicon atoms.

These are, for example, octamethylcyclotetrasiloxane sold especially under the name Volatile Silicone 7207 by Union Carbide or Silbione 70045 V 2 by Rhône-Poulenc, decamethylcyclopentasiloxane sold under the name Volatile Silicone 7158 by Union Carbide, and Silbione 70045 V 5 by Rhône-Poulenc, and mixtures thereof.
Mention may also be made of cyclocopolymers of the dimethylsiloxane/methylalkylsiloxane type, such as Volatile Silicone FZ 3109 sold by the company Union Carbide, of chemical structure:

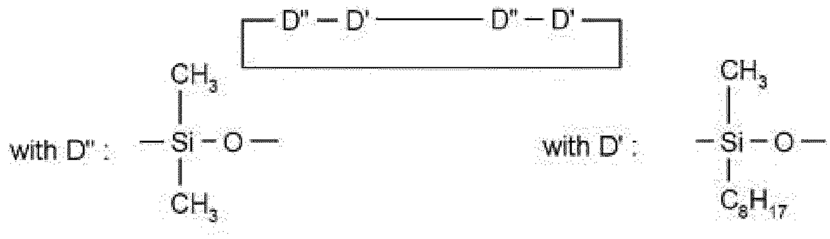

with D'' :

with D' :

Mention may also be made of mixtures of cyclic silicones with organosilicon compounds, such as the mixture of octamethylcyclotetrasiloxane and tetrakis(trimethylsilyl)-pentaerythritol (50/50) and the mixture of octamethylcyclotetrasiloxane and oxy-1,1'-bis(2,2,2',2',3,3'-hexatrimethylsilyloxy)-neopentane;

(ii) linear volatile silicones containing 2 to 9 silicon atoms and having a viscosity of less than or equal to $5\times10^{-6}$ $m^2/s$ at 25°C. An example is decamethyltetrasiloxane sold especially under the name SH 200 by the company Toray Silicone. Silicones belonging to this category are also described in the article published in Cosmetics and Toiletries,

Vol. 91, Jan. 76, pp. 27-32, Todd & Byers Volatile Silicone Fluids for Cosmetics.

Non-volatile silicones and more particularly polyalkylsiloxanes, polyarylsiloxanes, polyalkylarylsiloxanes, silicone gums and resins, and polyorganosiloxanes modified with organofunctional groups, and mixtures thereof, are preferably used.

These silicones are more particularly chosen from polyalkylsiloxanes, among which mention may be made mainly of polydimethylsiloxanes containing trimethylsilyl end groups (Dimethicone according to the CTFA name) having a viscosity of from $5\times10^{-6}$ to 2.5 $m^2/s$ at 25°C and preferably $1\times10^{-5}$ to 1 $m^2/s$. The viscosity of the silicones is measured, for example, at 25°C according to standard ASTM 445 Appendix C.

Among these polyalkylsiloxanes, mention may be made, in a non-limiting manner, of the following commercial products:

- the Silbione oils of the 47 and 70 047 series or the Mirasil oils sold by Rhône-Poulenc, for instance the oil 70 047 V 500 000;
- the oils of the Mirasil series sold by the company Rhône-Poulenc; the oils of the 200 series from the company Dow Corning, such as, more particularly, DC200 with a viscosity of 60 000 cSt (centistokes);
- the Viscasil oils from General Electric and certain oils of the SF series (SF 96, SF 18) from General Electric.

Mention may also be made of polydimethylsiloxanes containing dimethylsilanol end groups (Dimethiconol according to the CTFA name) such as the oils of the 48 series from the company Rhône-Poulenc.

Mention may also be made of polydimethylsiloxanes containing aminoethyl aminopropyl and $\alpha,\omega$-silanol groups.

In this category of polyalkylsiloxanes, mention may also be made of the products sold under the names Abil Wax 9800 and 9801 by the company Goldschmidt, which are poly($C_1$-$C_{20}$)alkylsiloxanes.

The polyalkylarylsiloxanes are particularly chosen from linear and/or branched polydimethylmethylphenylsiloxanes and polydimethyldiphenylsiloxanes with a viscosity of from $1\times10^{-5}$ to $5\times10^{-2} m^2/s$ at 25°C.

Among these polyalkylarylsiloxanes, examples that may be mentioned include the products sold under the following names:

- the Silbione oils of the 70 641 series from Rhône-Poulenc;
- the oils of the Rhodorsil 70 633 and 763 series from Rhône-Poulenc;
- the oil Dow Corning 556 Cosmetic Grade Fluid from Dow Corning;
- silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

The silicone gums that may be present in the composition according to the invention are especially polydiorganosiloxanes having high number-average molecular masses of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Mention may be made more particularly of the following products:

polydimethylsiloxane gums,
polydimethylsiloxane/methylvinylsiloxane gums,
poly[(dimethylsiloxane)/(vinylhydrogenosiloxane)] gums,
poly[(dimethylsiloxane)/(divinylhydrogenosiloxane)] gums,
polydimethylsiloxane/diphenylsiloxane gums,
polydimethylsiloxane/phenylmethylsiloxane gums,
polydimethylsiloxane/diphenylsiloxane/methylvinylsiloxane gums.

Products that may be used more particularly are the following mixtures:

- mixtures formed from a polydimethylsiloxane hydroxylated at the end of the chain (known as dimethiconol according to the nomenclature of the CTFA dictionary) and from a cyclic polydimethylsiloxane (known as cyclomethicone according to the nomenclature of the CTFA dictionary), such as the product Q2 1401 sold by the company Dow Corning;
- mixtures formed from a polydimethylsiloxane gum with a cyclic silicone, such as the product SF 1214 Silicone Fluid from the company General Electric, this product being an SF 30 gum corresponding to a dimethicone, having a

number-average molecular weight of 500 000, dissolved in the oil SF 1202 Silicone Fluid corresponding to decamethylcyclopentasiloxane;

- mixtures of two PDMSs with different viscosities, and more particularly of a PDMS gum and a PDMS oil, such as the product SF 1236 from the company General Electric. The product SF 1236 is a mixture of a gum SE 30 defined above with a viscosity of 20 $m^2/s$ and of an oil SF 96 with a viscosity of $5\times10^{-6}$ $m^2/s$. This product preferably comprises 15% of gum SE 30 and 85% of an oil SF 96.

The organopolysiloxane resins that may be present in the composition according to the invention are crosslinked siloxane systems containing the following units: $R_2SiO_{2/2}$, $R_3SiO_{1/2}$, $RSiO_{3/2}$ and $SiO_{4/2}$ in which R represents a hydrocarbon group containing 1 to 16 carbon atoms or a phenyl group. Among these products, those that are particularly preferred are the ones in which R denotes a $C_1$-$C_4$ lower alkyl radical, more particularly methyl, or a phenyl radical.

Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethyl siloxysilicate type resins sold in particular under the names X22-4914, X21-5034 and X21-5037 by the company Shin-Etsu.

The organomodified silicones that may be present in the composition according to the invention are silicones as defined above and comprising in their structure one or more organofunctional groups attached via a hydrocarbon-based radical.

Among the organomodified silicones, mention may be made of polyorganosiloxanes comprising:

- polyethyleneoxy and/or polypropyleneoxy groups optionally comprising C6-C24 alkyl groups, such as the oxyethylenated and oxypropylenated poly(methyllauryl/methylsiloxane) sold under the name Dow Corning 5200 Formulation Aid by the company Dow Corning (INCI: Lauryl PEG/PPG-18/18 methicone), the products known as dimethicone copolyol sold by the company Dow Corning under the name DC 1248 or the oils Silwet L 722, L 7500, L 77 and L 711 by the company Union Carbide, and the (C12)alkylmethicone copolyol sold by the company Dow Corning under the name Q2 5200;
- thiol groups, such as the products sold under the names GP 72 A and GP 71 from Genesee;
- alkoxylated groups, such as the product sold under the name Silicone Copolymer F755 by SWS Silicones and Abil Wax 2428, 2434 and 2440 by the company Goldschmidt; hydroxylated groups, such as the polyorganosiloxanes containing a hydroxyalkyl function, described in French patent application FR-A-85/16334 ;
- acyloxyalkyl groups, for instance the polyorganosiloxanes described in patent US-A-4 957 732
- anionic groups of the phosphate or carboxylic acid type, for instance in the products described in patent EP 186 507 from the company Chisso Corporation, or of the alkylcarboxylic type, such as those present in the product X-22-3701E from the company Shin-Etsu; 2-hydroxyalkyl sulfonate; 2-hydroxyalkyl thiosulfate such as the products sold by the company Goldschmidt under the names Abil S201 and Abil S255;
- hydroxyacylamino groups, for instance the polyorganosiloxanes described in patent application EP 342 834. Mention may be made, for example, of the product Q2-8413 from the company Dow Corning.

Among the organomodified silicones, mention may also be made of amino silicones.

The term "amino silicone" means any polyaminosiloxane, i.e. any polysiloxane comprising at least one primary, secondary or tertiary amine function or a quaternary ammonium group.

Preferably, the amino silicone(s) used in the cosmetic composition according to the present invention are chosen from:

(a) the compounds corresponding to formula (V) below:

$$(R^1)_a(T)_{3-a}\text{-Si}[OSi(T)_2]_n\text{-}[OSi(T)_b(R^1)_{2-b}]_m\text{-}OSi(T)_{3-a}\text{-}(R^1)_a)a \qquad (V)$$

in which:

T is a hydrogen atom or a phenyl, hydroxyl (-OH) or C1-C8 alkyl radical, and preferably methyl, or a C1-C8 alkoxy, preferably methoxy,

a denotes the number 0 or an integer from 1 to 3, and preferably 0,

b denotes 0 or 1, and in particular 1,

m and n are numbers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10;

$R^1$ is a monovalent radical of formula $-C_qH_{2q}L$ in which q is a number from 2 to 8 and L is an optionally quaternized amino group chosen from the following groups:

$-N(R^2)-CH_2-CH_2-N(R^2)_2$;

$-NR^2_2$; $-N^+R^2)_3Q^-$;

$-N^-(R^2)(H)_2Q^-$ ;

$-N^+(R^2)_2HQ^-$;

$-N(R^2)-CH_2-CH_2-N^+(R^2)(H)_2Q^-$,

in which $R^2$ denotes a hydrogen atom, a phenyl, a benzyl or a saturated monovalent hydrocarbon-based radical, for example a $C_1$-$C_{20}$ alkyl radical, and $Q^-$ represents a halide ion, for instance fluoride, chloride, bromide or iodide.

In particular, the amino silicones corresponding to the definition of formula (V) are chosen from the compounds corresponding to formula (VI) below:

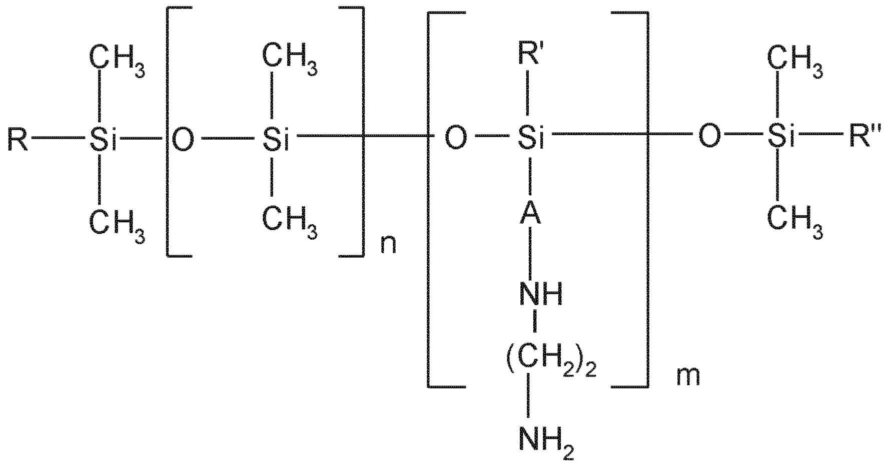

(VI)

in which R, R' and R", which may be identical or different, denote a $C_1$-$C_4$ alkyl radical, preferably $CH_3$; a $C_1$-$C_4$ alkoxy radical, preferably methoxy; or OH; A represents a linear or branched, $C_3$-$C_8$ and preferably $C_3$-$C_6$ alkylene radical; m and n are integers dependent on the molecular weight and whose sum is between 1 and 2000.

According to a first possibility, R, R' and R", which may be identical or different, represent a $C_1$-$C_4$alkyl or hydroxyl radical, A represents a $C_3$ alkylene radical and m and n are such that the weight-average molecular mass of the compound is between 5000 and 500 000 approximately. Compounds of this type are referred to in the CTFA dictionary as "amodimethicones".

According to a second possibility, R, R' and R", which may be identical or different, represent a $C_1$-$C_4$ alkoxy or hydroxyl radical, at least one of the radicals R or R" is an alkoxy radical and A represents a $C_3$ alkylene radical. The hydroxy/alkoxy mole ratio is preferably between 0.2/1 and 0.4/1 and advantageously equal to 0.3/1. Moreover, m and n are such that the weight-average molecular mass of the compound is between 2000 and $10^6$. More particularly, n is between 0 and 999 and m is between 1 and 1000, the sum of n and m being between 1 and 1000.

In this category of compounds, mention may be made, inter alia, of the product Belsil® ADM 652 sold by Wacker.

According to a third possibility, R and R", which are different, represent a $C_1$-$C_4$ alkoxy or hydroxyl radical, at least one of the radicals R or R" is an alkoxy radical, R' represents a methyl radical and A represents a $C_3$ alkylene radical. The hydroxy/alkoxy mole ratio is preferably between 1/0.8 and 1/1.1 and advantageously equal to 1/0.95. Moreover, m and n are such that the weight-average molecular mass of the compound is between 2000 and 200 000. More particularly, n is between 0 and 999 and m is between 1 and 1000, the sum of n and m being between 1 and 1000.

More particularly, mention may be made of the product Fluid WR® 1300 sold by Wacker.

According to a fourth possibility, R and R" represent a hydroxyl radical, R' represents a methyl radical and A is a $C_4$-$C_8$ and preferably $C_4$ alkylene radical. Moreover, m and n are such that the weight-average molecular mass of the compound is between 2000 and $10^6$. More particularly, n is between 0 and 1999 and m is between 1 and 2000, the

sum of n and m being between 1 and 2000.

A product of this type is especially sold under the name DC 28299 by Dow Corning.

Note that the molecular mass of these silicones is determined by gel permeation chromatography (ambient temperature, polystyrene standard; μ styragem columns; eluent THF; flow rate 1 mm/m; 200 μl of a solution containing 0.5% by weight of silicone in THF are injected, and detection is performed by refractometry and UV-metry).

A product corresponding to the definition of formula (V) is in particular the polymer known in the CTFA dictionary (7th edition, 1997) as "trimethylsilyl amodimethicone", corresponding to formula (VII) below:

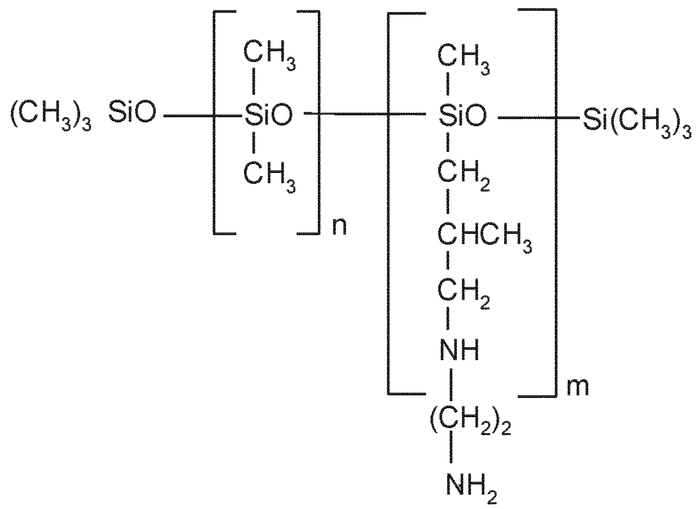

(VII)

in which n and m have the meanings given above in accordance with formula (V) or (VI).

Such compounds are described, for example, in EP 0 095 238 ; a compound of formula (VII) is sold, for example, under the name Q2-8220 by the company OSI.

(b) the compounds corresponding to formula (VIII) below:

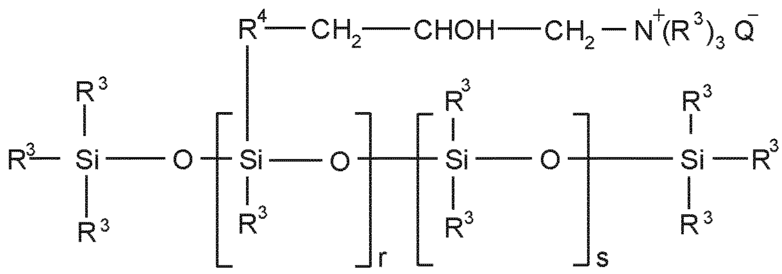

(VIII)

in which:

- $R^3$ represents a $C_1$-$C_{18}$ monovalent hydrocarbon-based radical, and in particular a $C_1$-$C_{18}$ alkyl or $C_2$-$C_{18}$ alkenyl radical, for example methyl;
- $R^4$ represents a divalent hydrocarbon-based radical, especially a $C_1$-$C_{18}$ alkylene radical or a divalent $C_1$-$C_{18}$, and for example $C_1$-$C_8$, alkylenoxy radical;
- $Q^-$ is a halide ion, in particular chloride;
- r represents a mean statistical value from 2 to 20 and in particular from 2 to 8;
- s represents a mean statistical value from 20 to 200 and in particular from 20 to 50.

Such compounds are described more particularly in patent US-4 185 087.

A compound falling within this class is the product sold by the company Union Carbide under the name Ucar Silicone ALE 56.

(c) the quaternary ammonium silicones of formula (IX):

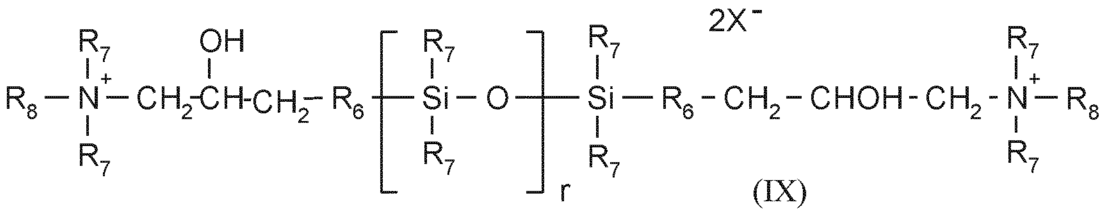

(IX)

in which:

$R_7$, which may be identical or different, represent a monovalent hydrocarbon-based radical containing from 1 to 18 carbon atoms, and in particular a $C_1$-$C_{18}$ alkyl radical, a $C_2$-$C_{18}$ alkenyl radical or a ring comprising 5 or 6 carbon atoms, for example methyl;
$R_6$ represents a divalent hydrocarbon-based radical, especially a $C_1$-$C_{18}$ alkylene radical or a divalent $C_1$-$C_{18}$, and for example $C_1$-$C_8$, alkylenoxy radical linked to the Si via an SiC bond;
$R_8$, which may be identical or different, represent a hydrogen atom, a monovalent hydrocarbon-based radical containing from 1 to 18 carbon atoms, and in particular a $C_1$-$C_{18}$ alkyl radical, a $C_2$-$C_{18}$ alkenyl radical or a radical -$R_6$-$NHCOR_7$;
$X^-$ is an anion such as a halide ion, especially chloride, or an organic acid salt (acetate, etc.);
r represents a mean statistical value from 2 to 200 and in particular from 5 to 100;
These silicones are described, for example, in patent application EP-A 0 530 974.

(d) the amino silicones of the following formula:

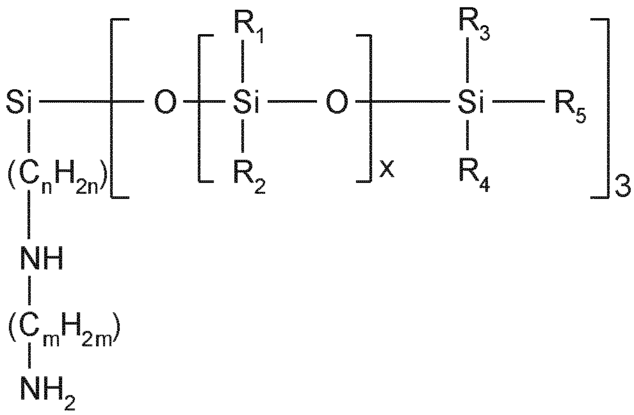

in which:

$R_1$, $R_2$, $R_3$ and $R_4$, which may be identical or different, denote a $C_1$-$C_4$ alkyl radical or a phenyl group,
$R_5$ denotes a $C_1$-$C_4$ alkyl radical or a hydroxyl group,
n is an integer ranging from 1 to 5,
m is an integer ranging from 1 to 5, and

in which x is chosen such that the amine number is between 0.01 and 1 meq/g.

The silicone(s) that are particularly preferred are polysiloxanes containing amine groups such as the silicones of formula (VI) or the silicones of formula (VII).

When these compounds are used, one particularly advantageous embodiment involves their combined use with cationic and/or nonionic surfactants.

By way of example, use may be made of the product sold under the name Cationic Emulsion DC 929 by the company Dow Corning, which comprises, besides amodimethicone, a cationic surfactant comprising a mixture of products corresponding to formula (X):

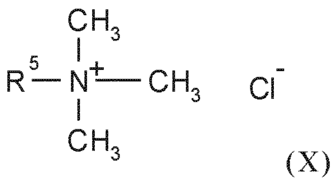

(X)

in which $R^5$ denotes $C_{14}$-$C_{22}$ alkenyl and/or alkyl radicals derived from tallow fatty acids, and known under the CTFA name "tallowtrimonium chloride", in combination with a nonionic surfactant of formula: $C_9H_{19}$-$C_6H_4$-$(OC_2H_4)_{10}$-OH, known under the CTFA name "Nonoxynol 10".

Use may also be made, for example, of the product sold under the name Cationic Emulsion DC 939 by the company Dow Corning, which comprises, besides amodimethicone, a cationic surfactant which is trimethylcetylammonium chloride and a nonionic surfactant of formula: $C_{13}H_{27}$-$(OC_2H_4)_{12}$-OH, known under the CTFA name "trideceth-12".

Another commercial product that may be used according to the invention is the product sold under the name Dow Corning Q2 7224 by the company Dow Corning, comprising, in combination, the trimethylsilyl amodimethicone of formula (C) described above, a nonionic surfactant of formula: $C_8H_{17}$-$C_6H_4$-$(OCH_2CH_2)_{40}$OH, known under the CTFA name "octoxynol-40", a second nonionic surfactant of formula: $C_{12}H_{25}$-$(OCH_2$-$CH_2)_6$-OH, known under the CTFA name "isolaureth-6", and propylene glycol.

Among all the silicones that may be present in the composition according to the invention, it is preferred to choose the silicone(s) from among non-volatile silicones and more particularly polyalkylsiloxanes, polyarylsiloxanes, polyalkylarylsiloxanes, silicone gums, polyorganosiloxanes modified with organofunctional groups chosen from amino silicones, and silicones comprising polyethyleneoxy and/or polypropyleneoxy groups, and also mixtures thereof.

Preferably, the silicone(s) that may be used in the cosmetic composition according to the present invention are chosen from amino silicones.

The silicone(s) that may be used in the cosmetic composition according to the invention may be present in a content ranging from 0.01% to 10% by weight, preferably in a content ranging from 0.05% to 5% by weight and better still in a content ranging from 0.1% to 2% by weight relative to the total weight of the composition.

According to one embodiment, the composition according to the invention is free of silicones.

The cosmetic composition according to the invention may also comprise one or more non-silicone fatty substances.

The term "fatty substance" means an organic compound that is insoluble in water at ordinary room temperature (25°C) and at atmospheric pressure (760 mmHg) (solubility of less than 5%, preferably 1% and even more preferentially 0.1%). In addition, the fatty substances are generally soluble in organic solvents under the same temperature and pressure conditions, for instance chloroform, ethanol, benzene, liquid petroleum jelly or decamethylcyclopentasiloxane.

Preferably, the non-silicone fatty substance(s) are chosen from fatty alcohols, fatty acids, esters of fatty acids and/or of fatty alcohols, and non-silicone animal, plant, mineral or synthetic oils other than the abovementioned esters.

For the purposes of the present invention, the term "fatty alcohol" means any saturated or unsaturated, linear or branched alcohol containing at least 8 carbon atoms.

The fatty alcohol may have the structure R-OH in which R denotes a saturated or unsaturated, linear or branched radical containing from 8 to 40 and preferably from 8 to 30 carbon atoms; R preferably denotes a $C_8$-$C_{40}$ and preferably $C_{12}$-$C_{24}$ alkyl or a $C_8$-$C_{40}$ and preferably $C_{12}$-$C_{24}$ alkenyl group. R may be substituted with one or more hydroxyl groups and especially with one or two hydroxyl groups.

Examples that may be mentioned include lauryl alcohol, cetyl alcohol, stearyl alcohol, oleyl alcohol, behenyl alcohol, linoleyl alcohol, undecylenyl alcohol, palmitoleyl alcohol, arachidonyl alcohol and erucyl alcohol, and mixtures thereof.

The fatty alcohol may represent a mixture of fatty alcohols, which means that several species of fatty alcohol may coexist, in the form of a mixture, in a commercial product.

Advantageously, the fatty alcohol is solid or pasty at a temperature of 25°C. For the purposes of the present invention, the term "fatty alcohol that is solid or pasty at 25°C" means a fatty alcohol that has a viscosity, measured with a rheometer (for example an R600 rheometer) at a shear rate of 1 $s^{-1}$, of greater than or equal to 1 Pa.s.

Preferably, the fatty alcohols used in the cosmetic composition according to the invention are cetylstearyl alcohol, stearyl alcohol and cetyl alcohol.

The fatty acids may be chosen from acids of formula RCOOH, in which R is a saturated or unsaturated, linear or branched radical preferably comprising from 7 to 39 carbon atoms.

Preferably, R is a $C_7$-$C_{29}$ alkyl or $C_7$-$C_{29}$ alkenyl group and better still a $C_{12}$-$C_{24}$ alkyl or $C_{12}$-$C_{24}$ alkenyl group. R may be substituted with one or more hydroxyl groups and/or one or more carboxyl groups.

The fatty acid may be chosen in particular from lauric acid, oleic acid, palmitic acid, linoleic acid, myristic acid and stearic acid. In order to be considered as a fatty acid, the fatty acid must not be in soap form, i.e. it must not be salified.

As non-silicone oils that may be used in the composition of the invention, examples that may be mentioned include:

- hydrocarbon-based oils of animal origin, such as perhydrosqualene;
- triglycerides of plant or synthetic origin, such as liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stéarineries Dubois or those sold under the names Miglyol® 810, 812 and 818 by the company Dynamit Nobel, jojoba oil and shea butter oil;
- linear or branched hydrocarbons of mineral or synthetic origin, containing more than 16 carbon atoms, such as volatile or non-volatile liquid paraffins, and derivatives thereof, petroleum jelly, liquid petroleum jelly, polydecenes, and hydrogenated polyisobutene such as Parleam®;
- partially hydrocarbon-based fluoro oils; fluoro oils that may also be mentioned include perfluoromethylcyclopentane and perfluoro-1,3-dimethylcyclohexane, sold under the names Flutec® PC1 and Flutec® PC3 by the company BNFL Fluorochemicals; perfluoro-1,2-dimethylcyclobutane; perfluoroalkanes such as dodecafluoropentane and tetradecafluorohexane, sold under the names PF 5050® and PF 5060® by the company 3M, or bromoperfluorooctyl sold under the name Foralkyl® by the company Atochem; nonafluoromethoxybutane and nonafluoroethoxyisobutane; perfluoromorpholine derivatives such as 4-trifluoromethyl perfluoromorpholine sold under the name PF 5052® by the company 3M.

As regards the esters of a fatty acid and/or of a fatty alcohol, which are advantageously different than the triglycerides mentioned above, mention may be made especially of esters of saturated or unsaturated, linear or branched $C_1$-$C_{26}$ aliphatic mono- or polyacids and of saturated or unsaturated, linear or branched $C_1$-$C_{26}$ aliphatic mono- or polyalcohols, the total carbon number of the esters more particularly being greater than or equal to 10.

Among the monoesters, mention may be made of dihydroabietyl behenate; octyldodecyl behenate; isocetyl behenate; cetyl lactate; $C_{12}$-$C_{15}$ alkyl lactate; isostearyl lactate; lauryl lactate; linoleyl lactate; oleyl lactate; (iso)stearyl octanoate; isocetyl octanoate; octyl octanoate; cetyl octanoate; decyl oleate; isocetyl isostearate; isocetyl laurate; isocetyl stearate; isodecyl octanoate; isodecyl oleate; isononyl isononanoate; isostearyl palmitate; methylacetyl ricinoleate; myristyl stearate; octyl isononanoate; 2-ethylhexyl isononate; octyl palmitate; octyl pelargonate; octyl stearate; octyldodecyl erucate; oleyl erucate; ethyl and isopropyl palmitates, 2-ethylhexyl palmitate, 2-octyldecyl palmitate, alkyl myristates such as isopropyl, butyl, cetyl, 2-octyldodecyl, myristyl or stearyl myristate, hexyl stearate, butyl stearate, isobutyl stearate; dioctyl malate, hexyl laurate, 2-hexyldecyl laurate.

Still within the context of this variant, esters of $C_4$-$C_{22}$ dicarboxylic or tricarboxylic acids and of $C_1$-$C_{22}$ alcohols and esters of monocarboxylic, dicarboxylic or tricarboxylic acids and of $C_2$-$C_{26}$ dihydroxy, trihydroxy, tetrahydroxy or pentahydroxy alcohols may also be used.

Mention may be made especially of: diethyl sebacate; diisopropyl sebacate; diisopropyl adipate; di-n-propyl adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates.

Among the esters mentioned above, it is preferred to use ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, butyl stearate, isobutyl stearate; dioctyl malate, hexyl laurate, 2-hexyldecyl laurate, isononyl isononanoate or cetyl octanoate.

The composition may also comprise, as fatty ester, sugar esters and diesters of $C_6$-$C_{30}$ and preferably $C_{12}$-$C_{22}$ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose (or saccharose), glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated $C_6$-$C_{30}$, and preferably $C_{12}$-$C_{22}$ fatty acids. If they are unsaturated, these compounds may have one to three conjugated or unconjugated carbon-carbon double bonds.

The esters according to this variant may also be selected from monoesters, diesters, triesters, tetraesters and

polyesters, and mixtures thereof.

These esters may be, for example, oleates, laurates, palmitates, myristates, behenates, cocoates, stearates, linoleates, linolenates, caprates and arachidonates, or mixtures thereof such as, especially, oleopalmitate, oleostearate and palmitostearate mixed esters.

More particularly, use is made of monoesters and diesters and especially sucrose, glucose or methylglucose monooleates or dioleates, stearates, behenates, oleopalmitates, linoleates, linolenates and oleostearates.

An example that may be mentioned is the product sold under the name Glucate® DO by the company Amerchol, which is a methylglucose dioleate.

Examples of esters or mixtures of esters of sugar and of fatty acid that may also be mentioned include:

the products sold under the names F160, F140, F110, F90, F70 and SL40 by the company Crodesta, respectively denoting sucrose palmitostearates formed from 73% monoester and 27% diester and triester, from 61% monoester and 39% diester, triester and tetraester, from 52% monoester and 48% diester, triester and tetraester, from 45% monoester and 55% diester, triester and tetraester, from 39% monoester and 61% diester, triester and tetraester, and sucrose monolaurate;

the products sold under the name Ryoto Sugar Esters, for example referenced B370 and corresponding to sucrose behenate formed from 20% monoester and 80% di-triester-polyester;

the sucrose mono-dipalmito-stearate sold by the company Goldschmidt under the name Tegosoft® PSE.

Preferably, the cosmetic composition according to the invention comprises one or more fatty substances chosen from fatty alcohols.

The fatty substance(s) may be present in the cosmetic composition in a content ranging from 0.1% to 30% by weight and preferably in a content ranging from 0.5% to 10% by weight, relative to the total weight of the said composition.

The cosmetic composition may also contain one or more polymers, preferably nonionic or cationic polymers.

Preferably, the cosmetic composition according to the invention comprises one or more cationic polymers.

For the purposes of the present invention, the term "cationic polymer" denotes any polymer containing cationic groups and/or groups that can be ionized into cationic groups.

The cationic polymers that may be present in the composition according to the invention may be chosen from any of those already known per se as improving the cosmetic properties of the hair, i.e. especially those described in patent application EP-A-337 354 and in French patents FR-2 270 846 , 2 383 660, 2 598 611, 2 470 596 and 2 519 863.

The polymer(s) are chosen from non-silicone polymers.

The cationic polymers that are preferred are chosen from those containing units comprising primary, secondary, tertiary and/or quaternary amine groups, which may either form part of the main polymer chain or be borne by a side substituent directly attached thereto.

The cationic polymers used generally have a number-average molecular mass of between 500 and $5 \times 10^6$ approximately and preferably between $10^3$ and $3 \times 10^6$ approximately.

Among the cationic polymers that may be mentioned more particularly are polymers of the polyamine, polyaminoamide and polyquaternary ammonium type.

These are known products. They are described in particular in French patents 2 505 348 and 2 542 997. Among the said polymers, mention may be made of:

(1) Homopolymers or copolymers derived from acrylic or methacrylic esters or amides and comprising at least one of the units of formula (X), (XI), (XII) or (XIII) below:

(X)

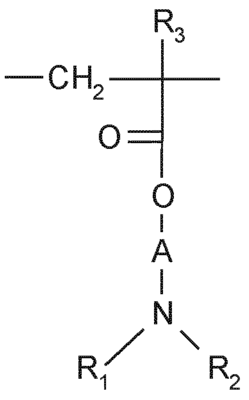

(XI)

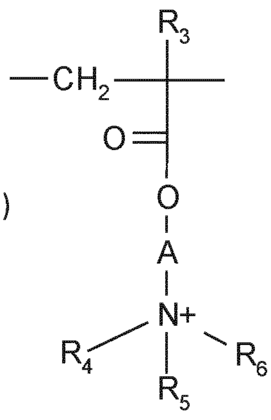

(XII)

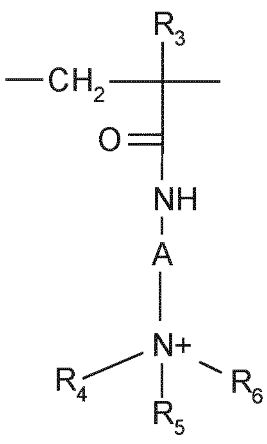

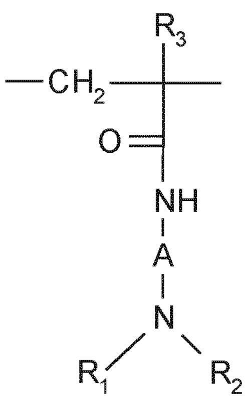

(XIII)

in which:

- $R_3$, which may be identical or different, denote a hydrogen atom or a $CH_3$ radical;
- A, which may be identical or different, represents a linear or branched alkyl group of 1 to 6 carbon atoms, preferably 2 or 3 carbon atoms, or a hydroxyalkyl group of 1 to 4 carbon atoms;
- $R_4$, $R_5$ and $R_6$, which may be identical or different, represent an alkyl group containing from 1 to 18 carbon atoms or a benzyl radical and preferably an alkyl group containing from 1 to 6 carbon atoms;
- $R_1$ and $R_2$, which may be identical or different, represent hydrogen or an alkyl group containing from 1 to 6 carbon atoms, and preferably methyl or ethyl;
- X denotes an anion derived from a mineral or organic acid, such as a methosulfate anion or a halide such as chloride or bromide.

Mention may be made in particular of the ethyltrimethylammonium methacrylate chloride homopolymer.

The polymers of family (1) can also contain one or more units derived from comonomers which may be chosen from the family of acrylamides, methacrylamides, diacetone acrylamides, acrylamides and methacrylamides substituted on the nitrogen with lower ($C_1$-$C_4$) alkyls, acrylic or methacrylic acids or esters thereof, vinyllactams such as vinylpyrrolidone or vinylcaprolactam, and vinyl esters.

Thus, among these polymers of family (1), mention may be made of:

- copolymers of acrylamide and of dimethylaminoethyl methacrylate quaternized with dimethyl sulfate or with a dimethyl halide, such as the product sold under the name Hercofloc by the company Hercules,
- copolymers of acrylamide and of methacryloyloxyethyltrimethylammonium chloride which are described, for example, in patent application EP-A-080976 and are sold under the name Bina Quat P 100 by the company Ciba Geigy,
- the copolymer of acrylamide and of methacryloyloxyethyltrimethylammonium methosulfate sold under the name Reten by the company Hercules,
- quaternized or non-quaternized vinylpyrrolidone/dialkylaminoalkyl acrylate or methacrylate copolymers, such as the products sold under the name Gafquat by the company ISP, such as, for example, Gafquat 734 or Gafquat 755, or alternatively the products known as Copolymer 845, 958 and 937. These polymers are described in detail in French

patents 2 077 143 and 2 393 573,

- dimethylaminoethyl methacrylate/vinylcaprolactam/vinylpyrrolidone terpolymers, such as the product sold under the name Gaffix VC 713 by the company ISP, vinylpyrrolidone/methacrylamidopropyldimethylamine copolymers sold in particular under the name Styleze CC 10 by ISP, and
- quaternized vinylpyrrolidone/dimethylaminopropylmethacryl-amide copolymers such as the product sold under the name Gafquat HS 100 by the company ISP, and
- the crosslinked polymers of methacryloyloxy($C_1$-$C_4$)alkyl tri($C_1$-$C_4$)alkylammonium salts, such as the polymers obtained by homopolymerization of quaternized dimethylaminoethyl methacrylate with methyl chloride, or by copolymerization of acrylamide with dimethylaminoethyl methacrylate quaternized with methyl chloride, the homo- or copolymerization being followed by crosslinking with an olefinically unsaturated compound, more particularly methylenebisacrylamide. A crosslinked acrylamide/methacryloyloxyethyltrimethylammonium chloride copolymer (20/80 by weight) in the form of a dispersion containing 50% by weight of the said copolymer in mineral oil can be used more particularly. This dispersion is sold under the name Salcare® SC 92 by the company Ciba. A crosslinked methacryloyloxyethyltrimethylammonium chloride homopolymer containing about 50% by weight of the homopolymer in mineral oil or in a liquid ester may also be used. These dispersions are sold under the names Salcare® SC 95 and Salcare® SC 96 by the company Ciba.

(2) Cellulose ether derivatives containing quaternary ammonium groups, described in French patent 1 492 597, and in particular polymers sold under the names Ucare Polymer "JR" (JR 400, JR 125 and JR 30M) or "LR" (LR 400 or LR 30M) by the company Amerchol. These polymers are also defined in the CTFA dictionary as quaternary ammoniums of hydroxyethylcellulose that have reacted with an epoxide substituted with a trimethylammonium group.

(3) Cellulose copolymers or cellulose derivatives grafted with a water-soluble monomer of quaternary ammonium, and described especially in US patent 4 131 576, such as hydroxyalkylcelluloses, for instance hydroxymethyl-, hydroxyethyl- or hydroxypropyl- celluloses grafted, in particular, with a methacryloylethyltrimethylammonium, methacrylamidopropyltrimethylammonium or dimethyldiallylammonium salt. The commercial products corresponding to this definition are more particularly the products sold under the names Celquat L 200 and Celquat H 100 by the company National Starch.

(4) The cationic guar gums described more particularly in US patents 3 589 578 and 4 031 307, such as guar gums containing cationic trialkylammonium groups. Guar gums modified with a 2,3-epoxypropyltrimethylammonium salt (e.g. chloride) are used, for example. Such products are sold in particular under the trade names Jaguar C13 S, Jaguar C 15, Jaguar C 17 or Jaguar C162 by the company Rhodia.

(5) Polymers formed from piperazinyl units and divalent alkylene or hydroxyalkylene radicals containing straight or branched chains, optionally interrupted with oxygen, sulfur or nitrogen atoms or with aromatic or heterocyclic rings, and also the oxidation and/or quaternization products of these polymers. Such polymers are described, in particular, in French patents 2 162 025 and 2 280 361.

(6) Water-soluble polyamino amides prepared in particular by polycondensation of an acidic compound with a polyamine; these polyamino amides can be crosslinked with an epihalohydrin, a diepoxide, a dianhydride, an unsaturated dianhydride, a bisunsaturated derivative, a bis-halohydrin, a bisazetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they contain one or more tertiary amine function(s), they can be quaternized. Such polymers are described, in particular, in French patents 2 252 840 and 2 368 508.

(7) Polyamino amide derivatives resulting from the condensation of polyalkylene polyamines with polycarboxylic acids followed by alkylation with difunctional agents. Mention may be made, for example, of adipic acid/dialkylaminohydroxy-alkyldialkylenetriamine polymers in which the alkyl radical contains from 1 to 4 carbon atoms and preferably denotes methyl, ethyl or propyl. Such polymers are especially described in French patent 1 583 363.

Among these derivatives, mention may be made more particularly of the adipic acid/dimethylamino-hydroxypropyl/diethylenetriamine polymers sold under the name Cartaretine F, F4 or F8 by the company Sandoz.

(8) Polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms. The mole ratio between the polyalkylene polyamine and the dicarboxylic acid is between 0.8: 1 and 1.4/ 1; the polyamino amide resulting therefrom is reacted with epichlorohydrin in a mole ratio of epichlorohydrin relative to the secondary amine group of the polyamino amide of between 0.5: 1 and 1.8/ 1. Such polymers are described in particular in US patents 3 227 615 and 2 961 347.

Polymers of this type are sold in particular under the name Hercosett 57 by the company Hercules Inc. or alternatively under the name PD 170 or Delsette 101 by the company Hercules in the case of the adipic acid/epoxypropyl/diethylenetriamine copolymer.

(9) Cyclopolymers of alkyldiallylamine or of dialkyldiallylammonium, such as the homopolymers or copolymers containing, as main constituent of the chain, units corresponding to formula (XIV) or (XV):

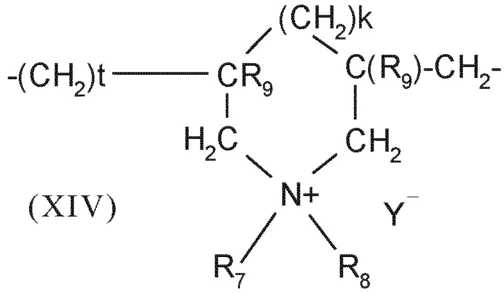

(XIV)

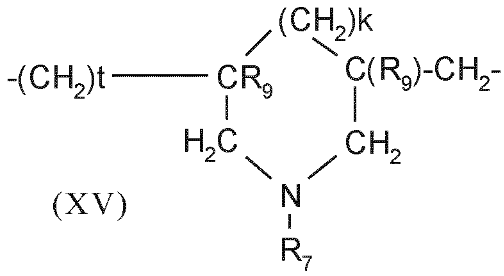

(XV)

in which formulae k and t are equal to 0 or 1, the sum k + t being equal to 1; R9 denotes a hydrogen atom or a methyl radical; R7 and R8 , independently of each other, denote an alkyl group containing from 1 to 6 carbon atoms, a hydroxyalkyl group in which the alkyl group preferably contains 1 to 5 carbon atoms, or a lower (C1-C4) amidoalkyl group, or R7 and R8 may denote, together with the nitrogen atom to which they are attached, heterocyclic groups, such as piperidyl or morpholinyl; R7 and R8 , independently of each other, preferably denote an alkyl group containing from 1 to 4 carbon atoms; Y is an anion such as bromide, chloride, acetate, borate, citrate, tartrate, bisulfate, bisulfite, sulfate or phosphate. These polymers are especially described in French patent 2 080 759 and in its Certificate of Addition 2 190 406.

Among the polymers defined above, mention may be made more particularly of the dimethyldiallylammonium chloride homopolymer sold under the name Merquat 100 by the company Nalco (and its homologues of low weight-average molecular mass) and the copolymers of diallyldimethylammonium chloride and of acrylamide, sold under the names Merquat 550 and Merquat 7SPR.

(10) The quaternary diammonium polymer containing repeating units corresponding to the formula:

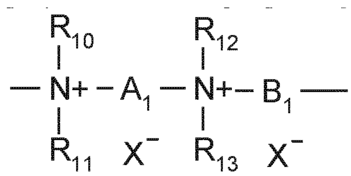

(XVI)

in which formula (XVI):

$R_{10}$, $R_{11}$, $R_{12}$ and $R_{13}$, which may be identical or different, represent aliphatic, alicyclic or arylaliphatic radicals containing from 1 to 6 carbon atoms or lower hydroxyalkylaliphatic radicals, or alternatively $R_{10}$, $R_{11}$, $R_{12}$ and $R_{13}$, together or separately, constitute, with the nitrogen atoms to which they are attached, heterocycles optionally containing a second heteroatom other than nitrogen, or alternatively $R_{10}$, $R_{11}$, $R_{12}$ and $R_{13}$ represent a linear or branched $C_1$-$C_6$ alkyl radical substituted with a nitrile, ester, acyl or amide group or a group $COOR_{14}D$ or $CONHR_{14}D$ where $R_{14}$ is an alkylene and D is a quaternary ammonium group;

$A_1$ and $B_1$ represent polymethylene groups containing from 2 to 8 carbon atoms, which may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and

$X^-$ denotes an anion derived from a mineral or organic acid;
$A_1$, $R_{10}$ and $R_{12}$ can form, with the two nitrogen atoms to which they are attached, a piperazine ring; in addition, if $A_1$ denotes a linear or branched, saturated or unsaturated alkylene or hydroxyalkylene radical, $B_1$ can also denote a group $-(CH_2)_nCODOC(CH_2)_n$ in which D denotes:

a) a glycol residue of formula: OZO, where Z denotes a linear or branched hydrocarbon-based radical or a group corresponding to one of the following formulae:

$-(CH_2-CH_2-O)x-CH_2-CH_2-$

$-[CH_2-CH(CH_3)-O]y-CH_2-CH(CH_3)-$

in which x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon-based radical, or alternatively the divalent radical

$-CH_2-CH_2-S-S-CH_2-CH_2-$;

d) a ureylene group of formula: -NH-CO-NH-.

Preferably, $X^-$ is an anion such as chloride or bromide.
These polymers generally have a number-average molecular mass of between 1000 and 100 000.
Polymers of this type are described in particular in French patents 2 320 330, 2 270 846, 2 316 271, 2 336 434 and 2 413 907 and US patents 2 273 780, 2 375 853, 2 388 614, 2 454 547 , 3 206 462 , 2 261 002 , 2 271 378, 3 874 870 , 4 001 432, 3 929 990, 3 966 904 , 4 005 193 , 4 025 617, 4 025 627, 4 025 653 , 4 026 945 and 4 027 020.
It is more particularly possible to use polymers that are formed from repeating units corresponding to formula (XVI) below:

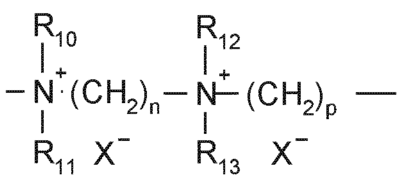
(XVI)

in which $R_{10}$, $R_{11}$, $R_{12}$ and $R_{13}$, which may be identical or different, denote an alkyl or hydroxyalkyl radical containing from 1 to 4 carbon atoms approximately, n and p are integers ranging from 2 to 8 approximately, and $X^-$ is an anion derived from a mineral or organic acid. Mention may be made in particular of Mexomer PO sold by the company Chimex.

(11) Polyquaternary ammonium polymers formed from repeating units of formula (XVII):

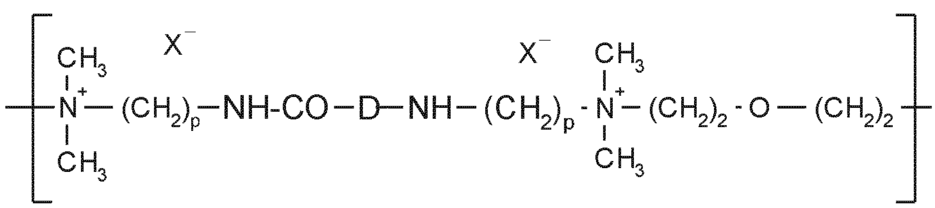
(XVII)

in which p denotes an integer ranging from 1 to 6 approximately, D may be nothing or may represent a group $-(CH_2)r-CO-$ in which r denotes a number equal to 4 or 7, and $X^-$ is an anion.
Such polymers may be prepared according to the processes described in US patents 4 157 388, 4 702 906 and 4 719 282. They are especially described in Patent application EP-A-122 324.
Among these polymers, examples that may be mentioned include the products Mirapol A 15, Mirapol AD1, Mirapol AZ1 and Mirapol 175 sold by the company Miranol.

(12) Quaternary polymers of vinylpyrrolidone and of vinylimidazole, for instance the products sold under the names Luviquat FC 905, FC 550 and FC 370 by the company BASF. These polymers may also comprise other monomers, for instance diallyldialkylammonium halides. Mention may be made in particular of the product sold under the name Luviquat Sensation by the company BASF.

(13) Polyamines such as Polyquart H sold by Henkel, which is given under the reference name Polyethylene glycol (15) Tallow Polyamine in the CTFA dictionary, or oxyethylenated (15 OE) coconut polyamines.

Other cationic polymers that may be used in the context of the invention are polyalkyleneimines, in particular polyethyleneimines, polymers containing vinylpyridine or vinylpyridinium units, condensates of polyamines and of epichlorohydrin, polyquaternary ureylenes and chitin derivatives.

Among all the cationic polymers that may be used in the context of the present invention, it is preferred to use polymers of families (1), (2), (3), (4), (9), (10) and (12).

Preferably, the cationic polymer(s) are chosen from cationic celluloses and cationic guar gums.

Preferably, the cationic polymer(s) are chosen from cationic celluloses, cationic guar gums and quaternary polymers of vinylpyrrolidone and of vinylimidazole optionally combined with other monomers.

The cationic polymer(s) that may be used in the composition according to the invention may be present in a content ranging from 0.01% to 10% by weight and preferably in a content ranging from 0.05% to 5% by weight, relative to the total weight of the cosmetic composition.

The compositions of the invention preferably comprise a cosmetically acceptable medium.

The term "cosmetically acceptable medium" means a medium that is compatible with keratin fibres, such as the hair.

The cosmetically acceptable medium is formed from water or from a mixture of water and one or more cosmetically acceptable solvents chosen from C1-C4 lower alcohols, such as ethanol, isopropanol, tert-butanol or n-butanol; polyols such as those of the invention, glycerol, propylene glycol, dipropylene glycol and polyethylene glycols; polyol ethers such as those of the invention, and mixtures thereof.

The composition according to the invention may comprise an amount of water which is greater than or equal to 5% by weight, relative to the total weight of the composition, preferably an amount of water greater than or equal to 20% by weight relative to the total weight of the composition.

Preferably, the amount of water in the composition according to the invention is less than or equal to 95% and preferentially less than or equal to 90% by weight relative to the total weight of the composition. The organic solvents may be present in a concentration ranging from 0.1% to 40% and better still from 1% to 20% by weight relative to the total weight of the composition.

The pH of the compositions according to the invention generally ranges from 3 to 11.

The composition according to the invention may also comprise one or more standard additives that are well known in the art, such as natural or synthetic thickeners or viscosity regulators; ceramides or pseudoceramides; sequestrants; solubilizers; proteins; reducing agents or antioxidants; oxidizing agents; vitamins or provitamins; cationic or amphoteric polymers; fragrances; pH stabilizers, preserving agents other than those of the invention; and permanent or temporary dyes such as natural or synthetic direct dyes (base or coupler), or mixtures thereof.

The thickener(s) may be chosen from cellulose-based thickeners, for example hydroxyethylcellulose, hydroxypropylcellulose and carboxymethylcellulose, guar gum and derivatives thereof, for example the hydroxypropyl guar sold by the company Rhodia under the reference Jaguar HP 105, gums of microbial origin, such as xanthan gum and scleroglucan gum, synthetic thickeners such as crosslinked acrylic acid or acrylamidopropanesulfonic acid homopolymers, for example Carbomer, nonionic, anionic, cationic or amphoteric associative polymers, such as the polymers sold under the names Pemulen TR1 or TR2 by the company Goodrich, Salcare SC90 by the company Ciba, Aculyn 22, 28, 33, 44 or 46 by the company Rohm & Haas, and Elfacos T210 and T212 by the company Akzo.

In particular, the thickener(s) that may be used in the cosmetic composition according to the invention are chosen from cellulose-based thickeners, in particular hydroxyalkylcelluloses. Preferably, the thickener(s) are chosen from hydroxyethylcellulose, hydroxypropylcellulose and carboxymethylcellulose, in particular hydroxyethylcellulose.

A person skilled in the art will take care to select the optional additives and the amount thereof such that they do not harm the properties of the compositions of the present invention.

These additives are generally present in the composition according to the invention in an amount ranging from 0 to 20% by weight relative to the total weight of the composition.

Preferably, the cosmetic composition according to the invention does not contain any preserving agents other than those of the invention.

The compositions in accordance with the invention may be used for conditioning keratin fibres, in particular the hair, for example as shampoos or hair conditioners.

Preferably, the compositions of the invention are rinse-out or leave-in hair conditioners.

The cosmetic compositions according to the invention may be in the form of creams, gels or lotions.

A subject of the invention also consists of the use of the cosmetic composition as described above for the cosmetic treatment, in particular the conditioning, of keratin fibres such as the hair.

Another subject of the present invention is a cosmetic process for treating keratin fibres, such as the hair, which consists in applying an effective amount of a composition as described above to the said fibres, and optionally in rinsing it out after an optional leave-on time.

In particular, 1 to 50 g and preferably 5 to 20 g of the cosmetic composition according to the invention may be applied to the keratin fibres.

The compositions described above may be used on any type of hair: light or dark hair, natural hair or hair that has undergone a cosmetic treatment such as permanent waving, dyeing, bleaching or relaxing.

The cosmetic composition according to the invention may be used on wet or dry hair. Preferably, the cosmetic composition is applied to clean hair.

Moreover, the leave-on time of the cosmetic composition on the hair may be between a few seconds and 30 minutes, preferably between 10 seconds and 15 minutes and even more preferentially between 1 minute and 10 minutes.

The application to the hair of the cosmetic composition according to the invention may be performed, for example, using a comb, a fine brush, a coarse brush or with the fingers.

The cosmetic composition is preferably rinsed out.

According to one particular embodiment of the invention, the application of the composition is followed by drying at room temperature or at a temperature above 40°C.

The drying may be performed immediately after the application or after a leave-on time that may range from 1 minute to 30 minutes.

Preferably, the hair is dried, in addition to using a supply of heat, with a flow of air. This flow of air during drying makes it possible to improve the individualization of the coating.

During drying, a mechanical action may be exerted on the locks, such as combing, brushing or by running the fingers through the hair.

The drying step of the process of the invention may be performed with a hood, a hair dryer or a straightening iron.

When the drying step is performed with a hood or a hair dryer, the drying temperature is between 40 and 110° and preferably between 50 and 90°.

When the drying step is performed with a straightening iron, the drying temperature is between 110 and 220° and preferably between 130 and 200°.

The examples below are given as illustrations of the present invention.

EXAMPLES

Example 1

1. Test compositions

A composition (A) according to the invention and a comparative composition (B) are prepared from the ingredients indicated in the table below, the amounts of which are expressed as weight percentages of active material relative to the total weight of the composition, unless otherwise indicated.

| Compositions | A (invention) | B (comparative) |
|---|---|---|
| Mixture of cetyl alcohol (>95%), stearyl alcohol and myristyl alcohol (Lanette 16 from Cognis) | 3.7 | 3.7 |
| Hydroxyethylcellulose (Natrosol 250 HHR from Aqualon) | 1 | 1 |
| Behenyltrimethylammonium chloride as a solution in isopropanol containing 80% AM (Genamin KDMP from Clariant) | 0.5 | 0.5 |
| Calcium chloride dihydrate | 4 | 4 |
| Phenoxyethanol | 0.7 | - |
| Caprylyl glycol | 0.3 | - |
| Water | qs 100% | qs 100% |

It is found that composition (A) according to the invention has a creamy, white, glossy, uniform appearance and is stable on storage. The microbiological protection over time of composition (A) is satisfactory.

2. Procedure:

On a panel of 20 women formed from 10 women with natural hair and 10 women with sensitized hair, 12 to 20 g of each of the compositions (A) and (B), as a function of the length of the hair, are applied. The term "sensitized hair" means hair that has undergone prior chemical or physical treatments.
After application, the hair is rinsed, combed and then dried by blow-drying or in the open air.
An expert then evaluates the cosmetic properties afforded by the cosmetic compositions (A) and (B) on wet hair and on dry hair once dried.
It is found that, on wet hair, hair treated with cosmetic composition (A) according to the invention is smooth, supple and fine.
Similarly, it is found that hair treated with cosmetic composition (A) according to the invention dries quickly.
In addition, it is observed that, on dry hair, hair treated with cosmetic composition (A) according to the invention is smooth, shiny, light and fine, and has a silky feel.
It is also found that when locks are treated with the cosmetic composition according to the invention (A), it is possible to control the volume of the locks at high humidity.

Example 2

Compositions (C)-(D) and (F) are not part of the invention. Composition (E) according to the invention is prepared from the ingredients indicated in the table below, the amounts of which are expressed as weight percentages of active material relative to the total weight of the composition, unless otherwise indicated.

| Compositions | C | D | E | F |
|---|---|---|---|---|
| Mixture of cetyl alcohol (>95%), stearyl alcohol and myristyl alcohol (Lanette 16 from Cognis) | 3.7 | 3.7 | 3.7 | 3.7 |
| Hydroxyethylcellulose (Natrosol 250 HHR from Aqualon) | 1 | 1 | 1 | 1 |
| Behenyltrimethylammonium chloride as a solution in isopropanol containing 80% AM (Genamin KDMP from Clariant) | 0.5 | 0.5 | 0.5 | 0.5 |
| Calcium gluconate | 8 | - | - | - |
| Calcium acetate | - | - | - | 4 |
| Calcium lactate | - | 6 | - | - |
| Calcium chloride dihydrate | - | - | 4.5 | - |
| Phenoxyethanol | 1 | 1 | - | 1 |
| Caprylyl glycol | 0.5 | 0.5 | - | 0.5 |
| Diethylene glycol monobenzyl ether | - | - | 1.5 | - |
| Hexylene glycol | - | - | 0.5 | - |
| Water qs | 100% | 100% | 100% | 100% |

## Claims

**1.** Cosmetic composition for treating keratin fibres, in particular human keratin fibres such as the hair, comprising:

- one or more polyol ethers containing one or more aromatic groups chosen from ethylene glycol monophenyl ether (or phenoxyethanol), ethylene glycol diphenyl ether, ethylene glycol monobenzyl ether, propylene glycol monophenyl ether, propylene glycol monobenzyl ether, diethylene glycol monophenyl ether, diethylene glycol monobenzyl ether, dipropylene glycol monophenyl ether and dipropylene glycol monobenzyl ether, and mixtures thereof, in a content ranging from 0.05 to 2% by weight relative to the total weight of the composition,
- one or more non-aromatic and non-etherified $C_6$-$C_8$ diols in a content ranging from 0.05 to 2% by weight relative to the total weight of the composition, and
- one or more hygroscopic water-soluble salts in a content ranging from 2 to 10% by weight relative to the total weight of the composition, relative to the total weight of the said cosmetic composition; the hygroscopic salt

being calcium chloride.

2. Cosmetic composition according to Claim 1, **characterized in that** the polyol ether containing one or more aromatic groups is phenoxyethanol.

3. Cosmetic composition according to any one of the preceding claims, **characterized in that** the non-aromatic and non-etherified $C_6$-$C_8$ diol(s) are chosen from, hexylene glycol, 1,2-hexanediol, 1,6-hexanediol and 1,2-octanediol (or caprylyl glycol), and mixtures thereof.

4. Cosmetic composition according to any one of the preceding claims, **characterized in that** the non-aromatic and non-etherified $C_6$-$C_8$ diol is caprylyl glycol.

5. Cosmetic composition according to any one of the preceding claims, **characterized in that** it also comprises one or more silicones and/or one or more non-silicone fatty substances chosen from fatty alcohols, fatty esters, fatty acids and non-silicone animal, plant, mineral or synthetic oils.

6. Cosmetic process for treating keratin fibres, in particular human keratin fibres such as the hair, **characterized in that** an effective amount of a composition according to any one of Claims 1 to 5 is applied to the said keratin fibres, and is optionally rinsed out after an optional leave-on time.

7. Use of the composition according to any one of Claims 1 to 5, for the conditioning of keratin fibres, in particular human keratin fibres such as the hair, in particular as a hair conditioner.

## Patentansprüche

1. Kosmetische Zusammensetzung zur Behandlung von Keratinfasern, insbesondere menschlichen Keratinfasern wie dem Haar, umfassend:

   - einen oder mehrere Polyolether mit einer oder mehreren aromatischen Gruppen, ausgewählt aus Ethylenglykolmonophenylether (oder Phenoxyethanol), Ethylenglykoldiphenylether, Ethylenglykolmonobenzylether, Propylenglykolmonophenylether, Propylenglykolmonobenzylether, Diethylenglykolmonophenylether, Diethylenglykolmonobenzylether, Dipropylenglykolmonophenylether und Dipropylenglykolmonobenzylether und Mischungen davon, in einem Gehalt im Bereich von 0,05 bis 2 Gew.-%, bezogen auf das Gesamtgewicht der Zusammensetzung,
   - ein oder mehrere nichtaromatische und nicht veretherte $C_6$-$C_8$-Diole in einem Gehalt im Bereich von 0,05 bis 2 Gew.-%, bezogen auf das Gesamtgewicht der Zusammensetzung, und
   - ein oder mehrere hygroskopische wasserlösliche Salze in einem Gehalt im Bereich von 2 bis 10 Gew.-%, bezogen auf das Gesamtgewicht der Zusammensetzung, bezogen auf das Gesamtgewicht der kosmetischen Zusammensetzung; wobei es sich bei dem hygroskopischen Salz um Calciumchlorid handelt.

2. Kosmetische Zusammensetzung nach Anspruch 1, **dadurch gekennzeichnet, dass** es sich bei dem Polyolether mit einer oder mehreren aromatischen Gruppen um Phenoxyethanol handelt.

3. Kosmetische Zusammensetzung nach einem der vorhergehenden Ansprüche, **dadurch gekennzeichnet, dass** das nichtaromatische und nicht veretherte $C_6$-$C_8$-Diol bzw. die nichtaromatischen und nicht veretherten $C_6$-$C_8$-Diole aus Hexylenglykol, 1,2-Hexandiol, 1,6-Hexandiol und 1,2-Octandiol (oder Caprylylglykol) und Mischungen davon ausgewählt ist bzw. sind.

4. Kosmetische Zusammensetzung nach einem der vorhergehenden Ansprüche, **dadurch gekennzeichnet, dass** es sich bei dem nichtaromatischen und nicht veretherten $C_6$-$C_8$-Diol um Caprylylglykol handelt.

5. Kosmetische Zusammensetzung nach einem der vorhergehenden Ansprüche, **dadurch gekennzeichnet, dass** sie außerdem ein oder mehrere Silikone und/oder eine oder mehrere Nicht-Silikon-Fettsubstanzen, die aus Fettalkoholen, Fettestern, Fettsäuren und tierischen, pflanzlichen, mineralischen oder synthetischen Nichtsilikonölen ausgewählt sind, umfasst.

6. Kosmetisches Verfahren zur Behandlung von Keratinfasern, insbesondere menschlichen Keratinfasern wie dem

Haar, **dadurch gekennzeichnet, dass** man auf die Keratinfasern eine wirksame Menge einer Zusammensetzung nach einem der Ansprüche 1 bis 5 aufbringt und nach einer fakultativen Einwirkungszeit gegebenenfalls ausspült.

7. Verwendung der Zusammensetzung nach einem der Ansprüche 1 bis 5 zum Konditionieren von Keratinfasern, insbesondere menschlichen Keratinfasern wie dem Haar, insbesondere als Haarkonditionierungsmittel.

**Revendications**

1. Composition cosmétique pour le traitement de fibres kératiniques, en particulier de fibres kératiniques humaines telles que des cheveux, comprenant

   - un ou plusieurs éthers de polyol contenant un ou plusieurs groupements aromatiques, choisis parmi le monophényl-éther d'éthylène-glycol (ou phénoxy-éthanol), le diphényl-éther d'éthylèneglycol, le monobenzyl-éther d'éthylèneglycol, le monophényl-éther de propy-lèneglycol, le monobenzyl-éther de propylèneglycol, le monophényl-éther de diéthylèneglycol, le monobenzyl-éther de diéthylèneglycol, le monophényl-éther de dipropylèneglycol et le monobenzyl-éther de dipropylèneglycol, et leurs mélanges, dans une proportion valant de 0,05 à 2 %, en poids rapporté au poids total de la composition,
   - un ou plusieurs diols en $C_6$-$C_8$, non aromatiques et non éthérifiés, dans une proportion valant de 0,05 à 2 %, en poids rapporté au poids total de la composition,
   - et un ou plusieurs sels hygroscopiques hydrosolubles, dans une proportion valant de 2 à 10 %, en poids rapporté au poids total de ladite composition cosmétique, lequel sel hygroscopique est du chlorure de calcium.

2. Composition cosmétique selon la revendication 1, **caractérisée en ce que** l'éther de polyol contenant un ou plusieurs groupements aromatiques est du phénoxy-éthanol.

3. Composition cosmétique selon l'une des revendications précédentes, **caractérisée en ce que** le ou les diols en $C_6$-$C_8$ non aromatiques et non éthérifiés sont choisis parmi l'hexylèneglycol, le 1,2-hexanediol, le 1,6-hexanediol et le 1,2-octanediol (ou caprylyl-glycol) et leurs mélanges.

4. Composition cosmétique selon l'une des revendications précédentes, **caractérisée en ce que** le diol en $C_6$-$C_8$ non aromatique et non éthérifié est du caprylyl-glycol.

5. Composition cosmétique selon l'une des revendications précédentes, **caractérisée en ce qu'**elle comprend en outre une ou plusieurs silicones et/ou un ou plusieurs corps gras non siliconés choisis parmi les alcools gras, esters gras, acides gras et huiles non siliconées animales, végétales, minérales ou synthétiques.

6. Procédé de traitement cosmétiques de fibres kératiniques, en particulier de fibres kératiniques humaines telles que des cheveux, **caractérisé en ce qu'**on applique sur lesdites fibres kératiniques, en une quantité efficace, une composition cosmétique conforme à l'une des revendications 1 à 5, qu'on élimine éventuellement par rinçage après un éventuel temps de pose.

7. Utilisation d'une composition conforme à l'une des revendications 1 à 5 pour le conditionnement de fibres kératiniques, en particulier de fibres kératiniques humaines telles que des cheveux, en particulier en tant qu'après-shampooing.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9501152 A
- JP 2003095891 A
- JP 2000191514 A
- JP 200247119 B
- JP 01233208 A
- US 4874554 A
- US 4137180 A
- FR 8516334 A
- US 4957732 A
- EP 186507 A
- EP 342834 A
- EP 0095238 A
- US 4185087 A
- EP 0530974 A
- EP 337354 A
- FR 2270846
- FR 2383660
- FR 2598611
- FR 2470596
- FR 2519863
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- US 3227615 A
- US 2961347 A
- FR 2080759
- FR 2190406
- FR 2320330
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- US 4157388 A
- US 4702906 A
- US 4719282 A
- EP 122324 A


### Non-patent literature cited in the description


- **M.R. PORTER.** Handbook of Surfactants. Blackie & Son, 1991, 116-178
- CTFA dictionary. 1993
- Walter Noll's Chemistry and Technology of Silicones. Academic Press, 1968
- Volatile Silicone Fluids for Cosmetics. Cosmetics and Toiletries. Todd & Byers, vol. 91, 27-32